**Europäisches Patentamt**

**European Patent Office**

**Office européen des brevets**

(19)

(11) **EP 1 133 994 A1**

(12) **EUROPEAN PATENT APPLICATION**

(43) Date of publication:
**19.09.2001 Bulletin 2001/38**

(51) Int Cl.$^7$: **A61K 38/17**, A61K 31/7088,
A61K 39/395, A61P 35/00

(21) Application number: **00105189.5**

(22) Date of filing: **11.03.2000**

(84) Designated Contracting States:
**AT BE CH CY DE DK ES FI FR GB GR IE IT LI LU
MC NL PT SE**
Designated Extension States:
**AL LT LV MK RO SI**

(71) Applicant: **Biognostik Gesellschaft für
biomelekulare Diagnostik mbH
37079 Göttingen (DE)**

(72) Inventors:
• **Schlingensiepen, Karl-Herman Dr.
37120 Bovenden - Lenglern (DE)**
• **Schlingensiepen, Reimar Dr.
37083 Göttingen (DE)**
• **Apfel, Rainer Dr.
93161 Sinzig (DE)**
• **Brysch, Wolfgang Dr.
37075 Göttingen (DE)**

• **Jachimczak, Piotr Dr.
97074 Würzburg (DE)**
• **Bogdahn, Ulrich Prof.
93055 Regensburg (DE)**

(74) Representative:
**Meyers, Hans-Wilhelm, Dr.Dipl.-Chem. et al
Patentanwälte
von Kreisler-Selting-Werner
Postfach 10 22 41
50462 Köln (DE)**

Remarks:
The sequence listing, which is published as annex
to the application documents, was filed after the date
of filing. The applicant has declared that it does not
include matter which goes beyond the content of the
application as filed.

(54) **A method for reversing the immunosuppressive effects of the Melanoma Inhibitory Activity
"MIA"**

(57)     A method for stimulating immune cells and/or
the immune system, and/or reducing invasion and/or
metastasis of tumor cells by inhibiting expression and/
or functional activity of "Melanoma Inhibitory Activity"
MIA.

**EP 1 133 994 A1**

Annex to the application documents - subsequently filed sequences listing

SEQUENZPROTOKOLL

<110> Biognostik Gesellschaft für biomolekulare Diagnost

<120> A method for reversing the immunosuppressive effects of
      the Melanoma Inhibitory Activity "MIA"

<130> EP00105189.5 Biognostik

<140> 00105189.5
<141> 2000-03-11

<160> 9

<170> PatentIn Ver. 2.1

<210> 1
<211> 15
<212> DNA
<213> Homo sapiens

<400> 1
gtcaggaatc ggcag                                                    15


<210> 2
<211> 16
<212> DNA
<213> Homo sapiens

<400> 2
cttggagaag acatac                                                   16


<210> 3
<211> 14
<212> DNA
<213> Homo sapiens

<400> 3
tgcctcccca gaag                                                     14


<210> 4
<211> 17
<212> DNA
<213> Homo sapiens

<400> 4
cactggcagt agaaatc                                                  17


<210> 5
<211> 15
<212> DNA
<213> Homo sapiens

<400> 5

gctcactggc agtag                                                                              15


<210> 6
<211> 14
<212> DNA
<213> Homo sapiens

<400> 6
atggtcagga atcg                                                                               14


<210> 7
<211> 16
<212> DNA
<213> Homo sapiens

<400> 7
gaatggtcag gaatcg                                                                             16


<210> 8
<211> 14
<212> DNA
<213> Homo sapiens

<400> 8
catcgtggac tgtg                                                                               14


<210> 9
<211> 459
<212> DNA
<213> Homo sapiens

<400> 9
ggcagggcca gcggtaggct gagctcactg gcagtagaaa tcccatttgt ctgtcttcac 60
atcgactttg ccaggtttca gggtctggtc ctctcggaca atgctactgg ggaaatagcc 120
caggcgagca gccagatctc catagtaatc tccctgaacg ctgcctcccc agaagagccg 180
cccacggccc ttcagcttgg agaagacata caccacttgg ccccggtgaa tggtcaggaa 240
tcggcagtcg ggggccatgt agtcctgaag ggccacagcc atggagatag ggtggctgca 300
ctcctggtcc gcacacagct tccggtcagc cagcttgggc ataggaccac ccctgacacc 360
aggtccggag aaggcagaca gcaagatgat gacaccaagg cacaccaggg accgggccat 420
cgtggactgt gagcaagaga gtgagcaagg gggtgctgg                                                   459

9

**Description**

The Polypeptide "Melanoma Inhibitory Activity", MIA, was discovered in 1989 as a factor that Inhibits growth of melanoma tumor cells. The antiprollferative action of MIA was also demonstrated In other tumor cells and Peripheral Blood Mononuclear Cells. Thus, CANCER RES. 49; 5358-63, Bogdahn et al, (1989) demonstrated a very strong tumor cell growth-inhibiting effect of a factor called melanoma inhibitory activity (MIA). Three active fraction pools, named MIA-I, MIA-II and MIA-III were identified. Tumor stem cell colony formation was reduced by an astonishing 99.89% e. g. by MIA-II.

CANCER RES 50; 6981-86, Weilbach et al. (1990) further demonstrated that MIA inhibits cell proliferation by prolonging of the S-Phase and arrest of the cells in the G2 compartment.

PROC.AACR 40; 79, Jachimczak et al. (1999) further extended these observations to Peripheral Blood Mononuclear Cells (PBMCs), although to only a slight degree: "IL-2-stimulated PBMC proliferation has been only slightly inhibited by addition of MIA".

CANCER RES, 54; 5695-5701, Blesch et al. (1994) identified MIA as a 131-ainino acid precursor, processed Into a mature 107-amino acid protein. This publication confirmed that MIA acts as a potent tumor cell growth inhibitor for malignant melanoma cell and further extended this observation to other neuroectodermal tumors and concluded that "... MIA ... might be attractive as a future antitumor therapeutical substance."

Controversial data were obtained regarding correlation of MIA expression with melanoma progression. CANCER RES. 57; 3149-53, Bosserhoff et al. (1997) and ANTICANCER RES. 19; 2691-3, Bosserhoff et al. (1997) found enhanced MIA levels In 13- 23% of stage I and II melanomas, but In 100% of stage III or stage IV disease.

In contrast, CANCER RES. 55; 6237-43, van Groningen et al. (1995) found MIA mRNA expression in non metastasising cell lines and an inverse correlation of MIA mRNA expression with pigmentation in melanoma metastasis lesions, but notably expression was found to be absent in highly metastasising cell lines. Furthermore, CLIN. CANCER RES. 5; 1099-105, Muhlbauer et al. (1997) concluded that "...MIA amplification seems to be of little value as a surrogate marker for clinical staging or the detection of metastatic disease."

Surprisingly while the above literature suggested that MIA had the potential as a therapeutic agent to treat melanoma patients it was now found that in contrast MIA is a potent immunosuppressive factor and that agents inhibiting expression and/or function of MIA have therapeutic potential for treatment of neoplasms and immunosuppression.

The present invention pertains to a method for stimulating immune cells and/or the immune system, and/or reducing invasion and/or metastasis of tumor cells by inhibiting expression and/or functional activity of "Melanoma Inhibitory Activity" MIA.

According to the invention the stimulation of the immune system is preferably achieved by inhibiting expression and/or functional activity of "Melanoma Inhibitory Activity" MIA in combination with enhancing expression in target cells and/or target pathogens of the molecules listed under a) to m);

alternatively by vaccination with DNA and/or RNA coding for all or part of the molecules listed under a) to m) and/or polypeptides contained in the molecules listed under a) to m);

by transfection of an organism and/or transfecting the target cells and/or target pathogens with genes coding for the molecules listed under a) to m);

by applying to an organism and/or to the target cells and/or target pathogens the molecules listed under a) to m);

and/ or by enhancing the synthesis and/or function of molecules stimulating and/or enhancing and/or upregulating and/or positively regulating the immune response with molecules including the molecules listed under a) to m), wherein

a) represents molecules selected from the group comprising chemokines, including lymphotactin and/or immune cell attracting factors;

b) represents elements selected from the group comprising viruses and/or parts of viruses, including adeno viruses, papilloma viruses, Epstein-barr-Viruses, viruses that are non-pathogenic including Newcastle-Disease virus, Cow-pox-virus;

c) represents molecules selected from the group comprising autologous and/or heterologous MHC-molecules;

d) represents molecules selected from the group comprising molecules involved in antigen processing;

e) represents molecules selected from the group comprising molecules involved in antigen presentation;

f) represents molecules selected from the group comprising molecules involved in mediating immune-cell effects;

g) represents molecules selected from the group comprising molecules involved in mediating immune cell cytotoxic effects;

h) represents molecules selected from the group comprising molecules involved in antigen transportation;

i) represents molecules selected from the group comprising co-stimulatory molecules;

j) represents molecules selected from the group comprising peptides enhancing recognition by immune cell and/or cytotoxic effects of immune cells;

k) represents molecules selected from the group comprising peptides containing one or more amino acids differing between a protein in the target cell from the other cells within an organism including, but not limited to antigens, specific for melanoma cells and/or melanocytes and/or breast cells and/or breast cancer cells; according to the invention the Inhibition of the syntheses and/or function of MIA is achieved by using molecule of group I) wherein

l) represents molecules selected from the group comprising the peptides according to j) being

peptides containing one or more mutations and/or amino acid substitutions of the ras protein amino, the p53 protein, the EGF receptor protein, fusion peptides and/or fusion proteins, the retinoblastoma protein, peptides containing one or more mutations and/or amino acid substitutions and/or amino acid substitutions caused by gene rearrangements and/or gene translocations, peptides containing one or more mutations and/or amino acid substitutions of proteins coded by oncogenes and/or protooncogenes, proteins coded by anti-oncogenes and/or tumor suppressor genes;
peptides derived from proteins differing in the target cell by one or amino acids from the proteins expressed by other cells in tile same organism,
peptides derived from viral antigens and/or coded by viral nucleic acids,
peptides derived from proteins over expressed In the target cell compared to a normal cell
and combinations thereof

m) tumor cell extracts and/or tumor cell lysates and/or adjuvants.

In a preferred embodiment of the invention the Inhibition of the expression and/or functional activity of MIA is achieved by using at least one nucleic acid molecule, peptide, protein or low molecular weight substance. Preferably, the nucleic acid molecule is an oligo- or polynucleotide molecule, in particular an antisense molecule and/or ribozyme.
The inhibition of the synthesis and/or function of MIA Is preferably achieved by using at molecules comprising the following antisense sequences:

MIA-2841-W     GTC AGG AAT CGG CAG          (Seq. ID No 1)

MIA-1278-W     CTT GGA GAA GAC ATA C          (Seq. ID No 2)

MIA-2842-W     TGC CTC CCC AGA AG          (Seq. ID No 3)

or parts of the sequences having at least 8 nucleotides.
Preferably, the antisense and/or ribozyme molecule is derived by synthesising a sequence wholly or partially complementary to MIA mRNA and testing for inhibitory activity of MIA.
According to the Invention the antIsense and/or rIbozyme molecule Is for example integrated into a DNA delivery system, comprising viral and/or non-viral vectors together with lipids selected from the group of anionic lipids, cationic lipids, non-cationic lipids and mixtures thereof.

In a preferred embodiment of the invention the nucleic acid molecules contain flanking sequences and/or vector sequences and/or sequences enhancing the expression and/or transfection of the nucleic acid molecules. In a further preferred embodiment of the invention the nucleic acid molecules are part of one or more vectors and/or viral sequences and/or viral vectors.

According to the Invention it Is preferred that the antisense and/or ribozyme molecule is modified at one or more of the sugar moieties, the bases and/or the internucleotide linkages as well as the phosphate moieties. For example, the modification of the oligonuclcotides, ribozymes and/or nucleic acids comprises modifications such as phosphorothloate (S-ODN) intemucleotide linkages, methylphosphonate internucleotide linkages, phosphoramidate linkages, peptide linkages, 2'-O-alkyl modifications of the sugar, in particular methyl, ethyl, propyl, butyl and the like, 2'-methoxyethoxy modifications of the sugar and/or modifications of the bases. The various modifications may be combined in an oligo- or polynucleotld.

In a further preferred embodiment of the Invention the oligonucleotides, ribozymes and/or nucleic acids are coupled to or mixed with folic acid, hormones, steroid hormones such as oestrogene, progesterone, corticosteroids, mineral corticoids, peptides, proteoglycans, glycolipids, phospholipids and derivatives therof,

The peptide and/or protein which can be used in the method of the Invention can be obtained by screening an expression library and testing the expression products for inhibiting expression and/or functional activity of MIA.

Alternatively, a synthetic peptide and/or protein can be obtained by screening randomly synthesised peptides and/or polypeptides for Inhibiting expression and/or functional activity of MIA.

Preferably, according to the Invention the Inhibition of expression and/or functional activity of MIA as well as of the expression of the MIA gene and/or MIA mRNA is achieved by using an inhibitor of low molecular weight which can for example be obtained by combinatorial chemistry and testing the products for inhibiting expression and/or functional activity of MIA.

In a further embodiment of the invention, the inhibition of expression and/or functional activity MIA is achieved by using DNA or RNA derivatives including aptamers and/or spiegelmers that bind to MIA.

Inhibition of expression and/or functional activity of MIA is can also be achieved by using antibodies or antibody fragments, such as $F_{ab}$-fragments, single chain antibodies or combinations thereof. These molecules can be identified and obtained by screening antibody libraries and testing the expression products for inhibiting expression and/or functional activity of MIA.

Any of the foregoing elements, molecules or substances can be combined with an immunostimulatory agent, for example cytokines and/or inhibitors of the expression and/or function of Interleukin-10 and/or transforming growth factor beta (TGF-B) and/or Prostaglandin B2 and/or receptors for Prostaglandin E2 and/or inhibitors of VEGF.

The present invention is also concerned with a composition for the manufacturing of a medicament comprising a molecule or a combination of molecules which is able to inhibit the expression and/or functional activity of MIA.

The resulting medicament comprising an inhibitor of the expression and/or functional activity of MIA is also subject of the present invention. The medicament of the invention may be combined with an immunostimulatory agent.

Any of the foregoing elements, molecules or substances can be employed for the preparation of a medicament for the prevention or the treatment of neoplasms, infections and/or Immunosuppressive disorders.

Preferably, both, the inhibitor of MIA expression and/or functional activity is applied locally to a tumor or other pathologlcally affected site or organ and may also is applied systemically (e.g. i.v. or s.c. or orally).

The present invention is also related with the use of a method for stimulating the immune system by inhibiting expression and/or functional activity of "Melanoma Inhibitory Activity" (MIA) In combination with the use of methods and/or molecules enhancing the immune response against diseased cells or pathogens, methods and/or molecules enhancing immunogenicity of target cells and/or target pathogens and/or

immunostimulatory molecules, comprising cytokines including Interleukins, including IL-1, IL-2, IL-4, IL- 12, IL-18, such cytokines being applied systemically to an organism including man or being applied locally e.g. to certain regions or organs or parts of organ or compartments of a body

and/or

enhancing expression of cytokines In target cells or pathogens by stimulating their expression and/or by transfecting expression Systems into the target cell or target pathogen, capable of expressing these cytokines

and/or

chemokines attracting immune cells including lymphotactin, such chemokines being applied systemically to an organism including man or being applied locally e.g. to certain regions or organs or parts of organ or compartments of a body

and/or

enhancing expression of chemokines in target cells or pathogens by stimulating their expression and/or by transfecting expression systems into die target cell or target pathogen, capable of expressing these chemokines
and/or

peptides and/or DNA and/ or RNA molecules and or other antigens that are found in tumor cells and/or pathogens, but not in normal cells and/or

enhancing expression of peptides and/or antigens that are found in tumor cells and/or pathogens, but not in normal cells
and/or

tumor cell extracts and/or tumor cell lysates and/or adjuvants.

## Examples

Example 1

Allogenic anti-glioma LAK immune response was strongly inhibited by exogenous addition of MIA.

To study the effects of MIA upon cytotoxic T-lymphocytes (CTL) and Lymphoklne Activated Killer cells (LAK cells) a CARE-LASS assay has been employed (Lichtenfels, R., Biddison, W.E., Schulz, H., Vogt, A.B. and R. Martin. CARE-LASS (calcein-release assay), an improved fluorescence based test system to measure cytotoxic lymphocyte activity J. Immunol. Meth., 172: 227-239, 1994). Brlefly, glioma cells were harvested, washed In 5%FCS/PBS solution and resuspended at 10 Mio cells/ml in 5%FCS/FBS. Calcein-AM was added to a final concentration of 25 $\mu$M (Molecular Probes, USA). The cells were labeled for 30 min at 37 °C then washed twice In 5%FCS/PBS, adjusted to 1 Mio cells / ml and loaded into 96-well U-shaped microtiter plates at the final concentration of 0.1 Mio / 100 $\mu$L / 1 well (Nunc, Denmark). To measure cytotoxic activity of effector cells pretreated with MIA (f.c. 500 ng/ml), wells were loaded with 100 $\mu$L of CTL and LAK cells to produce the desired E:T ratios of 1:10 and 1:100. To measure spontaneous release and total release of calcein, wells were preloaded with 100 $\mu$L 5% FCS/PBS or 100 $\mu$L lysis buffer (50nM sodium-borate, 0.1% Triton® X 100, pH 9.0) respectively. After incubating the plate for 4 h at 37° C the supernatants (50 $\mu$L) were transferred into new wells and measured using an automated fluorescence scanner (Titertek Fluoroskan II, Germany). The percent cytotoxicity was determined from the following equation:

$$\frac{\text{F/CTL assay - F spontaneous release}}{\text{F total lysis - F spontanous release}} \times 100 = \text{\% cytotoxicity}$$

Results:

MIA inhibited autologous and allogenic LAK cytotoxicity against malignant glioma ceil lines (HTZ-17, -243, -374, -375) up to 40% compared to controls.

Example 2

Furthermore, inhibition of MIA synthesis in MIA-secreting melanoma cells enhanced autologous LAK and CTL activity.

To study the effects of MIA upon cytotoxic T-lymphocytes (CTL) and Lymphokine Activated Killer cells (LAK cells) a CARE-LASS assay has been employed as described above In Example 1.

Briefly, melanoma cells were harvested, washed in 5% FCS/PBS solution and resuspended at 10 Mio cells/ml in 5% PCS/PBS, Calcein-AM was added to a final concentration of 25 $\mu$M (Molecular Probes, USA). The cells were labeled for 30 min at 37 °C, then washed twice in 5% FCS/PBS, adjusted to 1 Mio cells / ml and loaded into 96-well U-shaped microtiter plates at the final concentration of 0.1 Mio / 100 $\mu$l/1 well (Nunc, Denmark). To measure cytotoxic activity of effector cells pretreated with MIA-antisense oligonucleotides (f.c. 1-5 $\mu$M), wells were loaded with 100 $\mu$l of CTL and LAK cells at E T ratios of 1:10 and 1:100. To measure spontaneous release and total release of calcein, wells were preloaded with 100 $\mu$l 5% FCS/PBS or 100 $\mu$l lysis buffer (50 nM sodium-borate, 0.1% Tritons X 100, pH 9.0) respectively. After incubating the plate for 4 h at 37 °C the supernatants (50 $\mu$L) were transferred into new wells and measured using an automated fluorescence scanner (Titertek Fluoroskan II, Germany). The cytotoxicity was determined according to the equation described in Example 1.

Results

Inhibition of endogenous MIA synthesis by specific phosphorothloate antisense oligonucleotides in human, MIA-secreting melanoma cell lines (GI and HW) was enhanced by up to 20 %autologous LAK cytotoxicity compared to untreated MIA-producing melanoma cell lines.

Active sequences inhibiting MIA expression were

| | | |
|---|---|---|
| MIA-2841-W | GTC AGG AAT CGG CAG | (Seq. ID No 1) |
| MIA-1278-W | CTT GGA GAA GAC ATA C | (Seq. ID No 2) |
| MIA-2842-W | TGC CTC CCC AGA AG | (Seq. ID No 3) |

Less active or inactive sequences inhibiting MIA expression were

| | | |
|---|---|---|
| MIA-2843-N | CAC TGG CAG TAG AAA TC | (Seq. ID No 4) |
| MIA-2844-N | GCT CAC TGG CAG TAG | (Seq. ID No 5) |
| MIA-0202-N | ATG GTC AGG AAT CG | (Seq. ID No 6) |
| MIA-1277-N | GAA TGG TCA GGA ATC G | (Seq. ID No 7) |
| MIA-2328-N | CAT CGT GGA CTG TG | (Seq. ID No 8) |

Example 3

Furthermore, peptides inhibiting MIA activity in MIA-secreting melanoma cells also enhanced autologous LAK activity by up to 30%.

Example 4

Inhibition of endogenous MIA synthesis by specific phosphorothioate antisense oligonucleotides in human, MIA-secreting melanoma as well as breast cancer cell lines strongly reduced their migration activity, as well as increasing their adhesion to matrices, both showing a strong Inhibitory effect of MIA inhibitors on tumor invasion and metastasis.

Example 5

Inhibition of endogenous MIA synthesis by specific phosphorothioate antisense oligonucleotides in human, MIA-secreting melanoma cell lines (GI and HW) in combination with application of the cytoklnes IL-12, IL-4, IL-18 and/ or antisense oligonucleotides specific for TGF-β increased the autologous LAK cytotoxicity even further compared to inhibition of endogenous MIA synthesis by specific phosphorothioate antisense oligonucleotides alone In MIA-producing tumor cell lines.

Example 6

Inhibition of endogenous MIA synthesis by a transfecting vector expressing the antisense sequence (SEQ. ID No 9):

```
GGCAGGGCCAGCGGTAGGCTGAGCTCACTGGCAGTAGAAATCCCATTTGTCT

GTCTTCACATCGACTTTGCCAGGTTTCAGGGTCTGGTCCTCTCGGACAATGC

TACTGGGGAAATAGCCCAGGCGAGCAGCCAGATCTCCATAGTAATCTCCCTG

AACGCTGCCTCCCCAGAAGAGCCGCCCACGGCCCTTCAGCTTGGAGAAGACA

TACACCACTTGGCCCCGGTGAATGGTCAGGAATCGGCAGTCGGGGGCCATGT

AGTCCTGAAGGGCCACAGCCATGGAGATAGGGTGGCTGCACTCCTGGTCCGC

ACACAGCTTCCGGTCAGCCAGCTTGGGCATAGGACCACCCCTGACACCAGGT

CCGGAGAAGGCAGACAGCAAGATGATGACACCAAGGCACACCAGGGACCGGG

CCATCGTGGACTGTGAGCAAGAGAGTGAGCAAGGGGGTGCTGG
```

or parts of this sequence in human MIA-secreting melanoma as well as breast cancer cell lines strongly reduced their tumor invasion and metastasis in scid-mice and nude mice.

Example 7

Inhibition of tumor invasion and metastasis was increased by a combination of inhibitors of MIA with inhibitors of VEGF or TGF-β.

**Claims**

1. A method for stimulating immune cells and/or the Immune system, and/or reducing invasion and/or metastasis of tumor cells by inhibiting expression and/or functional activity of "Melanoma Inhibitory Activity" MIA.

2. The method according to claim 1, wherein the Inhibition of the expression and/or functional activity of MIA is achieved by using at least one nucleic acid molecule or derivative thereof,

3. The method according to claim 2 wherein the at least one nucleic acid molecule is an oligonucleotide, an antisense nucleic acid and/or a ribozyme.

4. The method according to claim 1, wherein the Inhlbition of the synthesis and/or function of MIA is achieved using a molecule comprising the following antisense sequences:

   MIA-2841-W     GTC AGG AAT CGG CAG

   MIA-1278-W     CTT GGA GAA GAC ATA C

   MIA-2842-W     TGC CTC CCC AGA AG

   or parts of the sequences having at least 8 nucleotides.

5. The method according to claim 3 wherein the antisense and/or ribozyme molecule Is derived by synthesising a sequence wholly or partially complementary to MIA mRNA and testing for inhibitory activity of MIA.

6. The method according to claim 3 wherein the antisense and/or ribozyme molecule is integrated into a DNA delivery system comprising viral and/or non-viral vectors together with lipids selected from the group of anionic lipids, cationic lipids, non-cationic lipids and mixtures thereof.

7. The method according to claim 3 wherein the antisense and/or ribozyme molecule is modified at one or more of the sugar moieties, the bases and/or the internucleotide linkages and/or by coupling the antisense and/or ribozyme molecule to an enhancer of uptake and/or Inhibitory activity.

8. The method according to claim 1 wherein the inhibition of the expression and/or functional activity of MIA is achieved using peptides and/or proteins.

9. The method according to claim 8 wherein the peptide and/or protein is derived by screening an expression library and testing the expression products for inhibitory activity of MIA.

10. The method according to claim 8 wherein the peptide and/or protein is derived by screening randomly synthesised peptides and/or proteins for inhibitory activity of MIA.

11. The method according to claim 1, wherein the inhibition of the expression and/or the function activity of MIA is achieved using an inhibitor of low molecular weight.

12. The method according to claim 11 wherein the inhibitor of low molecular weight is obtainable by combinatorial chemistry and testing the products for inhibitory activity of MIA.

13. The method according to claim 1, wherein the inhibition the expression and/or functional activity of MIA is achieved using DNA or RNA derivatives Including aptamers and/or spiegelmers that bind to MIA.

14. The method according to claim 1 wherein the inhibition of MIA is achieved using antibodies or antibody fragments, such as $F_{ab}$-fragments, single chain antibody or combinations thereof.

**15.** The method according to claim 14 wherein the antibody or antibody fragments, such as $F_{ab}$-fragments, single chain antibody or combinations thereof are obtainable by screening antibody libraries and testing the expression products for inhibitory activity of MIA.

**16.** The method according to any of the claims 1 to 14 wherein additionally an immunostimulatory agent, such as cytokines and/or inhibitors of the expression and/or function of interleukin-10 and/or transforming growth factor beta (TGF-B) and/or Prostaglandin B2 and/or receptors for Prostaglandin E2 and/or inhibitors of VEGF.

**17.** A composition comprising a molecule or a combination of molecules for inhibiting the synthesis and/or function of MIA

**18.** A medicament comprising an inhibitor of the synthesis and/or function of MIA.

**19.** A medicament comprising an inhibitor of the synthesis and/or function of MIA combined with an immunostimulatory agent.

**20.** The use of the composition according to claim 17 for the preparation of a medicament for the prevention or the treatment of neoplasms, infections and/or immunosuppressive disorders.

**European Patent Office**

## PARTIAL EUROPEAN SEARCH REPORT

**Application Number**

which under Rule 45 of the European Patent Convention shall be considered, for the purposes of subsequent proceedings, as the European search report

EP 00 10 5189

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | BOSSERHOFF A -K ET AL: "MELANOMA-INHIBITING ACTIVITY, A NOVEL SERUM MARKER FOR PROGRESSION OF MALIGNANT MELANOMA" CANCER RESEARCH,US,AMERICAN ASSOCIATION FOR CANCER RESEARCH, BALTIMORE, MD, vol. 57, no. 15, 1 August 1997 (1997-08-01), pages 3149-3153, XP002060476 ISSN: 0008-5472 * the whole document * ---<br><br>-/-- | 1-20 | A61K38/17 A61K31/7088 A61K39/395 A61P35/00 |

TECHNICAL FIELDS SEARCHED (Int.Cl.7)

A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search

see sheet C

| Place of search | Date of completion of the search | Examiner |
|---|---|---|
| THE HAGUE | 22 December 2000 | Moreau, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03 82 (P04C07)

European Patent
Office

INCOMPLETE SEARCH
SHEET C

Application Number

EP 00 10 5189

Although claims 1-16 are directed to a method of treatment of the human/animal body (Article 52(4) EPC), the search has been carried out and based on the alleged effects of the compound/composition.

--------------------

Claim(s) searched completely:
     4

Claim(s) searched incompletely:
     1-3, 5-20

Reason for the limitation of the search:

Present claims 1-20 relate to a compound defined by reference to a desirable characteristic or property, namely the inhibition of the expression of the activity of MIA.

The claims cover all compounds or methods having this characteristic or property, whereas the application provides support within the meaning of Article 84 EPC and/or disclosure within the meaning of Article 83 EPC for only a very limited number of such compounds. In the present case, the claims so lack support, and the application so lacks disclosure, that a meaningful search over the whole of the claimed scope is impossible. Independent of the above reasoning, the claims also lack clarity (Article 84 EPC). An attempt is made to define the compound by reference to a result to be achieved. Again, this lack of clarity in the present case is such as to render a meaningful search over the whole of the claimed scope impossible. Consequently, the search has been carried out for those parts of the claims which appear to be clear, supported and disclosed, namely those parts relating to the compounds defined as antisense compounds on page 4 last paragraph and page 5 first line.

Present claims 2,3, 5-20 relate to an extremely large number of possible compounds. Support within the meaning of Article 84 EPC and/or disclosure within the meaning of Article 83 EPC is to be found, however, for only a very small proportion of the compounds claimed. In the present case, the claims so lack support, and the application so lacks disclosure, that a meaningful search over the whole of the claimed scope is impossible. Consequently, the search has been carried out for those parts of the claims which appear to be supported and disclosed, namely those parts relating to the antisenses compounds
indicated in the examples and closely related homologous compounds mentioned in the description at page 4, last paragraph and page 5 first line

**European Patent Office**    **PARTIAL EUROPEAN SEARCH REPORT**    Application Number

EP 00 10 5189

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| X | JACHIMCZAK PIOTR ET AL: "Immunosuppressive effects of melanoma-inhibiting activity (MIA) – a possible role in tumor-host interactions." PROCEEDINGS OF THE AMERICAN ASSOCIATION FOR CANCER RESEARCH ANNUAL, no. 41, March 2000 (2000-03), page 115 XP000971517 91st Annual Meeting of the American Association for Cancer Research.;San Francisco, California, USA; April 01-05, 2000, March, 2000 ISSN: 0197-016X * the whole document * | 1-20 | |
| A | EP 0 945 507 A (ROCHE DIAGNOSTICS) 29 September 1999 (1999-09-29) * the whole document * | 1-20 | TECHNICAL FIELDS SEARCHED (Int.Cl.7) |
| A | JACHIMCZAK P ET AL: "Modulation of peripheral blood mononuclear cell (PMBC) activation by adhesion-regulating protein MIA." PROCEEDINGS OF THE AMERICAN ASSOCIATION FOR CANCER RESEARCH ANNUAL, vol. 40, March 1999 (1999-03), page 79 XP000971516 90th Annual Meeting of the American Association for Cancer Research;Philadelphia, Pennsylvania, USA; April 10-14, 1999, March, 1999 ISSN: 0197-016X * the whole document * | 1-20 | |

EPO FORM 1503 03.82 (P04C10)

**ANNEX TO THE EUROPEAN SEARCH REPORT
ON EUROPEAN PATENT APPLICATION NO.**

EP 00 10 5189

This annex lists the patent family members relating to the patent documents cited in the above-mentioned European search report.
The members are as contained in the European Patent Office EDP file on
The European Patent Office is in no way liable for these particulars which are merely given for the purpose of information.

22-12-2000

| Patent document cited in search report | Publication date | Patent family member(s) | Publication date |
|---|---|---|---|
| EP 945507 A | 29-09-1999 | AU 3599199 A<br>WO 9950411 A | 18-10-1999<br>07-10-1999 |